# EUROPÄISCHE PATENTSCHRIFT

(11) **EP 0 548 685 B1**
(45) Veröffentlichungstag und Bekanntmachung des Hinweises auf die Patenterteilung: **17.07.1996**
(21) Anmeldenummer: 92121037.3
(22) Anmeldetag: 10.12.1992
(51) Int. Cl.: C07C 265/14, C07C 263/10, C07C 263/20, C07C 265/04

(54) **Verfahren zur Herstellung von Isocyanaten und Aufarbeitung des Rückstandes**
Method for preparing isocyanates and treatment of residue
Procédé de préparation des isocyanates et traitement du résidu

(30) Priorität: 23.12.1991 DE 4142769
(43) Veröffentlichungstag der Anmeldung: 30.06.1993
(73) Patentinhaber: BAYER AG, 51368 Leverkusen (DE)
(72) Erfinder: Arribas, Javier Calvo, Dipl.-Ing., E-43850 Cambrils, Tarragona (ES); Arribas, Ciriaco Domingo, E-43006 La Granja, Tarragona (ES); Rodriguez, Salvador Vidal, Dipl.-Ing., E-43080 Tarragona (ES)

(56) Entgegenhaltungen:
- EP-A- 0 269 218
- WO-A-90/09990
- FR-A- 2 155 362
- GB-A- 1 408 745
- US-A- 2 889 257

## Beschreibung

Die Erfindung betrifft ein Verfahren zur Herstellung von reinen destillierten Isocyanaten durch Umsetzung der entsprechenden Amine mit Phosgen in einem geeigneten Lösungsmittel und mehrstufige destillative Aufarbeitung in reine Isocyanate, reines Lösungsmittel und einen Anteil an Rückstand, wobei dieser Rückstand aufgearbeitet wird.

Die großtechnische Herstellung destillierter Isocyanate durch Umsetzung von Aminen mit Phosgen in Lösungsmitteln ist bekannt und in der Literatur ausführlich beschrieben (Ullmanns Encyklopädie der technischen Chemie, 4. Auflage, Band 13, Seite 347-357, Verlag Chemie, GmbH, D-6940 Weinheim, 1977). Wie in dieser Literatur weiterhin beschrieben ist, fällt bei der Herstellung reiner destillierter Isocyanate in den Destillationsprozessen ein Nebenproduktstrom an, der nach weitmöglicher destillativer Abtrennung von freien Isocyanaten als Rückstand entsorgt werden muß. Im Laborversuch ist es möglich, aus diesem Rückstandsstrom erhebliche Anteile an freiem Isocyanat weiter abzudestillieren. Allerdings wird der verbleibende Rückstand zu einer harten vernetzten Masse, die im technischen Prozeß nicht handhabbar ist. Im technischen Prozeß ist man daher gezwungen ca. 20-40% freies Isocyanat in dem zu entsorgenden Rückstandsstrom zu belassen, damit dieser Rückstandsstrom technisch handhabbar bleibt. Dies hat unter anderem den erheblichen Nachteil, daß einerseits Wertstoff verloren geht und andererseits mehr Abfall entsorgt werden muß.

Es sind deshalb Prozesse bekannt, um weiteres freies Isocyanat aus dem Rückstand zu gewinnen. Z.B. beschreibt das britische Patent 1 408 745 einen Extraktionsprozeß zur Gewinnung des freien Isocyanates. In EP 269 218 wird ein Aufarbeitungsprozeß durch Erhitzen dieses Rückstandsstroms mit einem Bad von geschmolzenem Metall oder Metallsalzen beschrieben. Aus DE 2 915 830 geht ein Destillationsprozeß dieses Rückstandsstroms in einem Fließbett hervor. All diese Prozesse erfordem aufwendige Apparate und/oder Hilfsstoffe, wobei Hilfsstoffe wie z.B. Metalle oder Metallsalze die spätere Entsorgung erheblich erschweren.

In US 2,889,257 wird ein Verfahren zur Wiedergewinnung von organischen Isocyanaten aus dem rohen Reaktionsgemisch beschrieben, bei dem ein noch Isocyanat-haltiger Rückstand erhalten wird, der als Suspension in Öl vorliegt. In einem zusätzlichen Verfahrensschritt muß dann der Feststoff von dem Öl bei erhöhter Temperatur durch Filtration, Sieben oder Zentrifugieren abgetrennt werden. Der verbleibende Reststoff ist nicht rieselfähig, sondern ölfeucht und erhält noch restliches Isocyanat.

Aufgabe war es daher, ein technisch einfaches und sicheres Verfahren zu einwickeln, das es erlaubt, weiteres freies Isocyanat aus den Rückständen des Destillationsprozesses der Isocyanatproduktion zu gewinnen, ohne daß die oben genannten Nachteile die Aufarbeitung ökologisch und ökonomisch verkomplizieren, und zwar so, daß der verbleibende nicht weiter aufarbeitbare Rückstand einfach und ohne Belastung der Umwelt zu entsorgen ist.

Diese Aufgabe konnte überraschenderweise mit dem erfindungsgemäßen Verfahren gelöst werden.

Gegenstand der Erfindung ist ein Verfahren zur Herstellung von reinen destillierten Isocyanaten durch Umsetzung der entsprechenden Amine mit Phosgen in einem geeigneten Lösungsmittel und mehrstufige destillative Aufarbeitung in reine Isocyanate, reines Lösungsmittel und einen Anteil an Rückstand, welches dadurch gekennzeichnet ist, daß der Rückstand in einen gerührten und beheizten Behälter geleitet wird, der mit Bitumen, die unter den Destillationsbedingungen inert sind, teilweise gefüllt ist, der Anteil an noch vorhandenen freien Isocyanaten aus dem Rückstand abdestilliert wird und der verbleibende Rückstand als rieselfähiger Feststoff ausgetragen, abgekühlt und gegebenenfalls nach Mahlung, verbrannt wird.

Überraschenderweise konnte mit Bitumen, unter den entsprechenden Destillationsbedingungen in Abhängigkeit von den Isocyanaten weiteres Isocyanat aus dem Rückstand abdestilliert werden. Dabei entstand unter den Reaktionsbedingungen eine krümelige, freifließende Masse, die praktisch frei an freiem Isocyanat ist. Vor einer Zwischenlagerung oder Weiterverarbeitung wird der Rückstand abgekühlt.

Das erfindungsgemäße Verfahren kann allgemein für die Herstellung verschiedener Isocyanate eingesetzt werden. Insbesondere werden mit diesem Verfahren folgende Isocyanate hergestellt: Toluylendiisocyanat, 1,6-Hexandiisocyanat, 1-Isocyanato-3,3,5-Trimethyl-5-Isocyanatomethylcyclohexan, 1,5-Naphthylendiisocyanat, Diisocyanatodiphenylmethan.

Der Rückstand, wie er bei der destillativen Aufarbeitung bei der Herstellung von Isocyanaten anfällt, enthält normalerweise 20-80 Gew.-% freies Isocyanat neben den polymeren Nebenprodukten, bevorzugt 40-60 Gew.-% freies Isocyanat.

Als Rührreaktor kann ein normaler Reaktor mit Heizmantel, der normalerweise für Hochdruckdampf geeignet ist, eingesetzt werden. Andere Heizmittel sind selbstverständlich auch einsetzbar. Der Reaktor hat außerdem einen großdimensionierten Brüdenaustritt für das abdestillierende Isocyanat sowie sonstige Einlauf- und Hilfseinrichtungen. Er ist mit einem randgängigen Rührer, wie er für hochviskose Produkte in der Literatur vorgesehen wird, ausgerüstet. Bevorzugt werden Ankerrührer oder Wendelrührer verwendet.

Der Reaktor wird bei einer Temperatur von 150-280°C, bevorzugt von 180-230°C, und unter einem Druck von 2-30 mbar, bevorzugt 10-20 mbar, betrieben. Vorzugsweise erfolgt die Destillation aus einem gerührten Sumpfbehälter, an dem sich ein geeignetes Kondensationssystem anschließt. Vor Ausführung der Destillation wird der Reaktor zuerst mit Bitumen in einer Menge von 1-20 Vol-%, bevorzugt 2-6 Vol-% seines Inhalts, beschickt.

Ganz besonders bevorzugt werden Bitumen, z.B. des Typs B 80, B 80 E, des Typs B 300 oder B 300 E eingesetzt (Kennzeichnung nach DIN 52 010). Nach Beendigung der Destillation kann der verbleibende Rückstand zur Abkühlung z.B. in ein gerührtes Wasserbad geleitet werden.

Die Erfindung soll anhand der nachfolgenden Beispiele näher erläutert werden.

### Beispiel 1

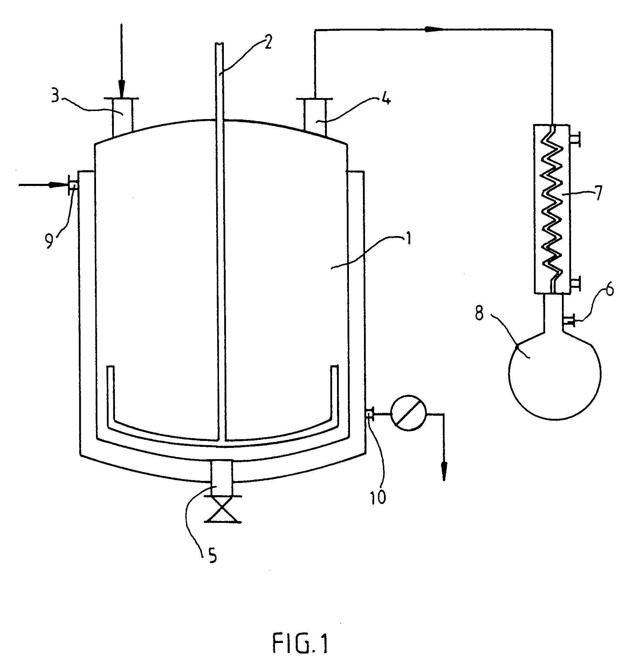

Man verwendet eine Apparatur nach Abbildung 1, bestehend aus einem Rührkessel (1) mit freitragendem, von oben betriebenem Ankerrührer (2). Der Rührkessel hat eine Höhe sowie einen Durchmesser von 50 cm (Kapazität 100 Liter) und ist mit einem Einlauf (3), Brüdenaustritt (4) und Bodenablaßstutzen (5) versehen. Er ist mit Hochdruckdampf (30 bar) über einen Heizmantel mit Dampfeintritt (9) und Kondensataustritt (10) beheizbar. Der Ankerrührer (2) ist von konventioneller Bauart mit einem Abstand von 10 mm zu den Wänden und dem Boden des Kessels und läuft mit 7 Umdrehungen pro Minute. Rührkessel (1) und Ankerrührer (2) sind aus nichtrostendem Stahl gefertigt.
Der Rührkessel (1) ist mit dem Brüdenaustritt (4) über einen wassergekühlten Brüdenkondensator (7) und den Gasaustritt (6) an eine Laborvakuumpumpe angeschlossen. Das anfallende Destillat wird in der Vorlage (8) gesammelt.

Für den Versuch wird aus der technischen Produktion von Toluylendiisocyanat (2,4/2,6 Isomerengemisch mit 80% 2,4-Toluylendiisocyanat) ein Rückstand der Zusammensetzung 41 Gew.-% 2,4- und 2,6-Toluylendiisocyanat, 39 Gew.-% polymerer Rückstand, 20 Gew.-% Lösungsmittel entnommen.

In den mit 30 bar Dampf vorgeheizten Rührkessel (1) werden 6 kg Bitumen des Typs B 80 vorgelegt. Man evakuiert den Kessel auf 15 mbar Vakuum und heizt ihn auf eine Innentemperatur von 200°C auf. Dann läßt man unter Rühren während 4 Stunden 7,5 kg/h des oben beschriebenen Rückstandes (insgesamt 30 kg) zulaufen. Anschließend wird das Gemisch 1 Stunde ohne weiteren Zulauf destilliert. Im Reaktor verbleibt ein trockener, sandartiger und leichtfließender Rückstand, der bei laufendem Rührer durch den Bodenstutzen (5) abgelassen werden kann.

In der Destillationsvorlage (8) erhält man ein Gemisch von 12,2 kg Toluylendiisocyanat und 5,8 kg Lösungsmittel. Der Reaktor (1) enthielt 17,8 kg Rückstand, der kein destillierbares Toluylendiisocyanat mehr aufwies.

### Beispiel 2

Beispiel 1 wird wiederholt mit dem Unterschied, daß 3,2 kg Bitumen des Typs B 80 vorgelegt werden und während 8 Stunden 7,5 kg pro Stunde Rückstand (insgesamt 60 kg) zulaufen.

Die Eigenschaften des erhaltenen Rückstands sind mit denen aus dem Beispiel 1 vergleichbar.

In der Destillationsvorlage (8) erhält man ein Gemisch von 24,5 kg Toluylendiisocyanat und 11,7 kg Lösungsmittel. Aus dem Reaktor (1) erhält man 26,6 kg Rückstand, der kein destillierbares Toluylendiisocyanat mehr enthält.

### Beispiel 3

Für den Versuch wird ein Rückstand aus der technischen Produktion von 1,6-Hexamethylendiisocyanat mit einer Zusammensetzung von 44 Gew.-% 1,6-Hexamethylendiisocyanat, 34 Gew.-% Rückstand und 22 Gew.-% Lösungsmittel eingesetzt. In der Apparatur von Beispiel 1 werden 5 kg Bitumen des Typs B 80 vorgelegt. Man evakuiert den Kessel bis 12 mbar und heizt ihn auf eine Innentemperatur von 190°C auf. Dann läßt man unter Rühren während 6 Stunden 8 kg/h des oben beschriebenen Rückstandes (insgesamt 48 kg) zulaufen.

Anschließend wird das Gemisch 90 Minuten ohne weiteren Zulauf destilliert. Im Reaktor verbleibt ein trockener, sandartiger und leichtfließender Rückstand, der bei laufendem Rührer durch den Bodenstutzen abgelassen werden kann.

In der Destillationsvorlage (8) erhält man ein Gemisch von 17,5 kg 1,6-Hexamethylendiisocyanat und 8,8 kg Lösungsmittel. Aus dem Reaktor (1) erhält man 21,4 kg Rückstand, der kein destillierbares 1,6-Hexamethylendiisocyanat mehr enthält.

## Patentansprüche

1. Verfahren zur Herstellung von reinen, destillierten Isocyanaten durch Umsetzung der entsprechenden Amine mit Phosgen in einem geeigneten Lösungsmittel und mehrstufige destillative Aufarbeitung der erhaltenen Isocyanatlösung in reine Isocyanate, reines Lösungsmittel sowie in einen Anteil an Rückstand, dadurch gekennzeichnet, daß der aus dem Destillationsprozeß erhaltene Rückstand in einen gerührten und auf 150-280°C beheizten Behälter bei einem Druck von 2-30 mbar geleitet wird, der mit Bitumen in einer Menge von 1-20 Vol.% seines Inhaltes gefüllt ist, der Anteil an noch im Rückstand vorhandenen freien Isocyanaten abdestilliert wird und der verbleibende Rückstand als rieselfähiger Feststoff ausgetragen, abgekühlt und gegebenenfalls nach Mahlung, einer Verbrennung zugeführt wird.

## Claims

1. Method for the preparation of pure, distilled isocyanates by reaction of the corresponding amines with phosgene in a suitable solvent and working up of the isocyanate solution obtained by multistage distillation into pure isocyanates, pure solvent and a proportion of residue, characterised in that the residue obtained from the distillation process is led into a stirred vessel heated to 150-280°C and at a pressure of 2-30 mbar, which vessel is filled with bitumen in a quantity of 1-20 vol.% of its capacity, the proportion of free isocyanates still present in the residue is distilled off and the residue remaining is discharged as a pourable solid substance, cooled and, optionally after grinding, is led away for combustion.

## Revendications

1. Procédé de préparation d'isocyanates distillés purs, par réaction des amines correspondantes avec du phosgène dans un solvant approprié et transformation par distillation en plusieurs étapes de la solution d'isocyanates obtenue en isocyanates purs, solvant pur, ainsi qu'en une part de résidu, caractérisé en ce que le résidu obtenu par le processus de distillation est introduit dans un récipient agité et chauffé à 150 - 280°C, sous une pression de 2 à 30 mbar, qui contient des bitumes dans une proportion de 1 à 20 % en volume de sa capacité, la quantité d'isocyanates libres encore présents dans le résidu est extraite par distillation et le résidu restant, sous forme de matière solide fluide, est évacué, refroidi et envoyé à une combustion, éventuellement après broyage.
